# FASCICULE DE BREVET EUROPEEN

(11) **EP 2 018 158 B1**
(45) Date de publication et mention de la délivrance du brevet: **24.04.2013**
(21) Numéro de dépôt: 07731453.2
(22) Date de dépôt: 14.05.2007
(51) Int. Cl.: A61K 9/20, A61K 9/28, A61K 31/223, A61P 1/12

(54) **NOUVELLE FORME D'ADMINISTRATION DU RACECADOTRIL**
NEUE FORM DER VERABREICHUNG VON RACECADOTRIL
NEW FORM OF ADMINISTRATION OF RACECADOTRIL

(30) Priorité: 15.05.2006 FR 0604302
(43) Date de publication de la demande: 28.01.2009
(73) Titulaire: BIOPROJET, 75003 Paris (FR)
(72) Inventeur: SCHWARTZ, Jean-Charles, 75014 Paris (FR); LECOMTE, Jeanne-Marie, 75003 Paris (FR)
(74) Mandataire: Blot, Philippe Robert Emile
(86) Numéro de dépôt international: PCT/FR2007/000814
(87) Numéro de publication internationale: WO 2007/132091

(56) Documents cités:
- EP-A- 1 563 848
- WO-A-01/97803
- WO-A2-01/97801
- SANKAR, D. GOWRI ET AL: "Spectrophotometric determination of famciclovir and racecadotril" ASIAN JOURNAL OF CHEMISTRY, vol. 17, no. 3, 2005, pages 2043-2045, XP008073284
- NICHOL G M ET AL: "EFFECT OF NEUTRAL ENDOPEPTIDASE INHIBITOR ON AIRWAY FUNCTION AND BRONCHIAL RESPONSIVENESS IN ASTHMATIC SUBJECTS", EUROPEAN JOURNAL OF CLINICAL PHARMACOLOGY, SPRINGER VERLAG, DE, vol. 42, no. 5, 1 January 1992 (1992-01-01), pages 491-494, XP008073220, ISSN: 0031-6970, DOI: 10.1007/BF00314856
- SALIBA FAOUZI ET AL: "Pathophysiology and therapy of irinotecan-induced delayed-onset diarrhea in patients with advanced colorectal cancer: A prospective assessment", JOURNAL OF CLINICAL ONCOLOGY - (PROC OF ASCO VOLUME 19 - 2000 ABSTR 2388), vol. 16, no. 8, 1 August 1998 (1998-08-01) , pages 2745-2751, XP008150349, ISSN: 0732-183X

## Description

La présente invention concerne une nouvelle formulation du racécadotril, son procédé de préparation et son utilisation dans le traitement des diarrhées.

Le racécadotril ou acétorphan est le composé racémique de formule (RS)-2-[[2-[(acétylsulfanyl)méthyl]-3-phénylpropanoyl]amino]acétate de benzyle.

C'est un puissant inhibiteur de l'enképhalinase, présentant une activité antisécrétrice intestinale originale, par protection des enképhalines endogènes contre leur dégradation. En améliorant l'activité biologique de ces neuropeptides au niveau des récepteurs opiacés delta, le racécadotril diminue les débits hydroélectrolytiques dans le lumen intestinal, ces débits étant, sinon, augmentés dans les maladies diarrhéiques d'origines diverses. De façon tout à fait intéressante, l'activité antidiarrhéique puissante du racécadotril est sélective en ce que l'hypersécrétion intestinale (ou la réabsorption électrolytique diminuée) caractérisant les diarrhées et responsable des états de déshydratation sévère est fortement diminuée sans modification du transit (Matheson A. J. & Noble S., Drugs 2000, 59, 829 ; Schwartz J.-C., Int. Antimicrob. Agents, 2000, 14, 81). Ce modèle, unique parmi les antidiarrhéiques, contribue au profil particulièrement intéressant du racécadotril, tel que cela a été désormais démontré dans de multiples essais cliniques et dans l'étude post-marketing, après utilisation par de millions de patients (Lecomte et al., Int. J. Antimicrob. Agents, 2000, 14, 81).

Dans les essais cliniques ainsi que dans la pratique courante, le racécadotril est généralement administré en gélules de 100 mg, prises trois fois par jour afin d'assurer une inhibition complète de la peptidase cible, et ce tout au long de la journée, sans interruption. Bien que très efficace, ce schéma d'administration ne favorise pas l'observance du traitement par le patient, notamment chez les patients qui ne souhaitent pas interrompre leur rythme habituel d'activité professionnelle. A cette fin, une prise deux fois par jour (b.i.d.) est préférable.

EP 1 563 848 A décrit des compositions comprenant du racécadotril et de l'ondansétron pour le traitement de la diarrhée. Ces compositions se présentent préférablement sous forme de comprimés, gélules ou poudre. L'administration s'effectue de 2 à 4 fois par jour.

Il est donc désirable d'améliorer l'observance du traitement au moyen d'une formulation du racécadotril permettant une administration deux fois par jour.

Néanmoins, dans la mesure où la demi-vie du racécadotrilate, le métabolite biologiquement actif de la prodrogue racécadotril, n'est que de 3-4 heures, il est nécessaire d'augmenter la biodisponibilité de la substance active pour permettre une inhibition de 24 heures de l'enképhalinase.

La biodisponibilité peut être modulée au moyen d'une formulation différente. Néanmoins, les comprimés sont considérés comme inférieurs aux gélules, les gélules étant généralement considérées comme la formulation permettant la biodisponibilité orale la plus importante.

Contre toute attente, et c'est là un des objets de la présente invention, les inventeurs ont découvert de façon inattendue que la formulation du racécadotril sous forme de comprimés permettait une biodisponibilité améliorée de la substance active, permettant ainsi une administration deux fois par jour.

Cette formulation permet donc une inhibition sur 24 heures de l'enzyme cible et une efficacité clinique améliorée comparée à la gélule habituellement utilisée, laquelle est administrée trois fois par jour.

Selon un premier objet, la présente invention concerne un comprimé de racécadotril comme défini dans la revendication 1. Selon un deuxième objet, la présente invention concerne un comprimé de racécadotril pour son utilisation pour le traitement de la diarrhée par administration deux fois par jour comme défini dans la revendication 7.

A priori, le racécadotril semble particulièrement inapproprié pour la préparation de comprimés. En effet, sa forme cristalline consistant en de longues aiguilles et sa très faible solubilité dans l'eau en rendent la compression directe difficile. De plus, de grandes quantités de racécadotril sont nécessaires, alors que il est généralement désirable d'obtenir un comprimé de petite taille (entre 10 et 15 mm de diamètre au plus) afin d'améliorer l'acceptabilité et l'observance du traitement par le patient. Par ailleurs, le racécadotril est insoluble dans l'eau, ce qui rend plus difficile une libération rapide de la molécule par désintégration du comprimé. Enfin, le racécadotril a un goût amer et une mauvaise odeur due à la présence de soufre dans la molécule ; il est donc nécessaire de mettre à disposition un comprimé masquant le goût et l'odeur.

Le racécadotril présente donc des propriétés particulièrement défavorables pour la formulation sous forme de comprimés pouvant être facilement produite à l'échelle industrielle et parfaitement acceptable et efficace pour les patients. Malgré ces préjugés, les présents inventeurs ont découvert que des comprimés comprenant du racécadotril répondent à ces différents critères.

Selon un premier aspect, les comprimés de racécadotril selon l'invention permettent une administration deux fois par jour (b.i.d.).

Selon un autre aspect, les comprimés de racécadotril selon l'invention permettent l'administration d'une dose de racécadotril comprise entre 170 et 180 mg par comprimé, de préférence environ 175 mg par comprimé.

Selon un autre aspect, les comprimés de racécadotril selon l'invention comprennent de 20 % à 50 % en poids de racécadotril.

Préférentiellement, lesdits comprimés sont constitués d'un noyau enrobé, ledit noyau comprenant du racécadotril. Le comprimé a un poids compris entre 350 et 600 mg.

Ledit noyau comprend, en plus du racécadotril, divers excipients habituellement utilisés, tels que :
- éventuellement une ou plusieurs charge(s) : telle que le monohydrate de lactose, permettant la préparation du comprimé par granulation humide, notamment le lactose de type "200 mesh" ou "110 mesh", présentant une granulométrie définie ou encore le monohydrate de lactose de type "Flowlac®" sous forme de poudre desséchée par nébulisation ; une autre charge possible est la cellulose microcristalline (par exemple de type Avicel® PH102). On peut également utiliser le mannitol ou sorbitol. A titre de charge, on préfère le monohydrate de lactose, type "200 mesh" ou "110 mesh", notamment « 110 mesh » ou de type "Flowlac®", ou encore la cellulose microcristalline (par exemple de type Avicel® PH102).
- éventuellement un ou plusieurs liant(s) tel que l'hydroxypropylcellulose ou la polyvidone; on préfère utiliser l'hydroxypropylcellulose, par exemple de type Klucel® EF, dans les granulés et la phase externe ;
- éventuellement un ou plusieurs désintégrant(s), tel que le carmellose calcium, l'amidon de maïs ou l'amidon prégélatinisé ; on préfère le carmellose calcium dans les granulés et la phase externe et/ou l'amidon prégélatinisé dans la phase externe;
- éventuellement un ou plusieurs lubrifiant(s), tel que le stéarate de magnésium, pour empêcher la masse de poudre compactée de coller à l'équipement; on préfère tout particulièrement le stéarate de magnésium dans la phase externe;

L'enrobage du noyau est constitué d'un ou plusieurs excipient(s) utilisés habituellement, de façon à masquer l'odeur de soufre et l'amertume de l'ingrédient actif. A titre de formulation d'enrobage, on peut citer les agents augmentant la viscosité, tels que l'alcool polyvinylique ; les opacifiants, tels que le dioxyde de titane ; les agents plastifiants hydrophiles, tels que les molécules de type Macrogol (par exemple Macrogol 3350) améliorant la flexibilité du film ; et les agents opacifiants colorants tels que le talc.

Pour préparer l'enrobage, ces divers ingrédients d'enrobage peuvent être mis en dispersion dans de l'eau purifiée. De façon particulièrement avantageuse, on peut utiliser le mélange prêt à l'emploi Opadry® comprenant :
- 40 % d'alcool polyvinylique,
- 25 % de dioxyde de titane,
- 20,2 % de Macrogol 3350, et
- 14,8 % de talc.

Selon un aspect préféré, les comprimés de racécadotril selon l'invention ont un noyau de composition suivante:
- 20 à 50 % de racécadotril ;
- 25 à 50 % de charge(s) ;
- 9 à 25 % de désintégrant(s) ;
- 2 à 10 % de liant(s) ;
- 0,5 à 5 % de lubrifiant(s) ;
   et plus préférentiellement :
- 20 à 50 % de racécadotril ;
- 20 à 40 % de monohydrate de lactose ;
- 7 à 15 % de carmellose calcium ;
- 2 à 10 % d'hydroxypropylcellulose ;
- 5 à 10 % de cellulose microcristalline ;
- 2 à 10 % d'amidon prégélatinisé ;
- 0,5 à 5 % de stéarate de magnésium.

Et encore plus préférentiellement, les noyaux des comprimés selon l'invention ont la composition suivante :
- 175 mg de racécadotril ;
- 144,1 mg de monohydrate de lactose ;
- 41 mg de carmellose calcium ;
- 18 mg d'hydroxypropylcellulose ;
- 32,5 mg de cellulose microcristalline;
- 25 mg d'amidon prégélatinisé ;
- 4,4 mg de stéarate de magnésium.

Selon un autre objet, la présente invention concerne également le procédé de préparation d'un comprimé de racécadotril selon l'invention, comprenant les étapes de :
1) la préparation du noyau comprenant le racécadotril, puis
2) l'enrobage dudit noyau.

La première étape comprend :
(i) la granulation
(ii) le séchage des granules obtenus ;
(iii) l'ajout et le mélange de la phase externe ; et
(iv) la compression du mélange final.

La granulation est réalisée par la méthode de granulation dite de granulation par voie humide. L'étape de granulation comprend :
a) la préparation d'un mélange de phase interne ;
b) l'ajout et le mélange du liquide de granulation à ladite phase interne.

La phase interne est généralement constituée, en sus du racécadotril, de charge(s) et désintégrant(s). Préférentiellement, la phase interne est constituée de racécadotril, monohydrate de lactose, carmellose calcium, et/ou éventuellement d'amidon de maïs.

Le liquide de granulation comprend le liant, préférentiellement l'hydroxypropylcellulose, et l'eau.

La phase externe est généralement constituée de lubrifiant(s), de charge(s), désintégrant(s) et/ou liant(s). Préférentiellement, la phase externe est constituée de monohydrate de lactose, cellulose microcristalline, carmellose calcium et stéarate de magnésium, et éventuellement l'amidon prégélatinisé, l'hydroxypropylcellulose.

Préférentiellement, les ingrédients de la phase interne sont ajoutés dans un agitateur tel que ceux habituellement utilisés, pendant une durée et à une vitesse suffisante permettant l'obtention d'un mélange homogène. On peut notamment citer l'agitateur de type Erweka ou Colette. Les durées de mélange sont comprises entre 1 et 20 minutes, à des vitesses de rotation comprises entre 150 et 500 rpm, de préférence environ 3 minutes à environ 200 rpm ou environ 10 minutes à environ 60 rpm.

Le liquide de granulation est préparé au moyen d'un agitateur à hélice en dispersant le liant dans l'eau jusqu'à l'obtention d'une solution limpide. Généralement, l'agitation est maintenue pendant 10 à 30 minutes, et les vitesses sont comprises entre 100 et 1000 rpm, de préférence environ 15 minutes à 500 rpm. Le liquide est ensuite ajouté au mélange précédent sous agitation ; de l'eau purifiée peut être ajoutée.

L'étape de séchage est réalisée avec un lit fluidisé (de type Glatt par exemple) ou dans un four à une température telle qu'une perte de poids au séchage de 1 % à 3 %, préférentiellement inférieure à 1,5 % est obtenue. Généralement, un séchage d'environ 20 heures à 40° est approprié. Le temps de séchage est ajusté de façon à permettre la calibration des granulés tout en limitant le taux de particules inclassables obtenues. Généralement, une humidité résiduelle de 1 % permet des résultats satisfaisants. Les granules secs obtenus sont généralement calibrés sur un tamis oscillant de 0,8 mm, tel que le tamis de type Frewitt.

Les ingrédients de la phase externe sont ajoutés aux granules et mélangés dans un agitateur, tel que ceux habituellement utilisés, pendant une durée et à une vitesse suffisante permettant l'obtention d'un mélange homogène. On peut notamment citer l'agitateur de type Turbula, Roehn ou Soneco. Les durées de mélange sont comprises entre 5 et 30 minutes, à des vitesses de rotation comprises entre 10 et 100 rpm, de préférence environ 15 minutes à environ 30 rpm ou environ 5 minutes à environ 10 rpm.

Préférentiellement, le stéarate de magnésium est préalablement tamisé sur un tamis de 0,315 mm ; il n'est ajouté qu'après mélange des autres ingrédients de la phase externe aux granules, puis le tout est mélangé pendant environ 1 à 5 minutes.

Pour la compression du mélange final, les paramètres d'équipement tels que la masse et la force de compression ainsi que la vitesse de compression peuvent être ajustés de façon à obtenir les comprimés désirés. La compression peut être effectuée sur tout type de machine permettant la préparation de comprimés, notamment les appareils de type Frogerais ou Courtoy R190 équipés de poinçon de type 11R11 ou 12R12. La dureté est fixée approximativement à 80N.

Les noyaux sont alors transférés sur une plateforme d'enrobage (par exemple de type Accela Cota) et chauffés (environ 40°C de préférence) pendant l'enrobage par pulvérisation de la suspension d'enrobage décrite plus haut. La pulvérisation est poursuivie jusqu'à un gain de poids d'environ 10 mg.

Les exemples suivants sont donnés à titre illustratif et non limitatif de la présente invention.

### Exemple 1

Les comprimés de racécadotril ont été préparés avec la composition suivante :

| **Phase interne** | | |
|---|---|---|
| | Racécadotril | 175 |
| | Monohydrate de lactose | 100 |
| | Carmellose calcium | 35 |

| **Liquide de granulation** | | |
|---|---|---|
| | Hydroxypropylcellulose | 12 |
| | Eau purifiée | sq |

| **Phase externe** | | |
|---|---|---|
| | Monohydrate de lactose | 44 |
| | Cellulose microcristalline | 32.5 |
| | Carmellose calcium | 6 |
| | Stéarate de magnésium | 2.5 |
| | Amidon prégélatinisé | 25 |
| | Hydroxypropylcellulose | 6 |

| **Enrobage** | | |
|---|---|---|
| | Opadry® White | 10 |
| | Eau purifiée | sq |

Les quantités indiquées ci-dessus sont données en mg.

Soit:
- 175 mg de racécadotril ;
- 144,1 mg de monohydrate de lactose ;
- 41 mg de carmellose calcium ;
- 18 mg d'hydroxypropylcellulose ;
- 32,5 mg de cellulose microcristalline;
- 25 mg d'amidon prégélatinisé ;
- 4,4 mg de stéarate de magnésium.
- 10 mg d'enrobage (Opadry® white)

### Exemple 2

Les comprimés de l'exemple 1 ont été préparés de la façon suivante : les ingrédients de la phase interne sont mélangés dans un agitateur de type Erweka pendant 3 minutes à 180 rpm. Une solution d'hydroxypropylcellulose à 7,5 % (poids/volume) est préparée dans l'eau afin d'améliorer les propriétés de liaison. Le liquide de granulation obtenu est ajouté au mélange de poudre obtenu précédemment, sous mélange à 180 rpm. Selon l'aspect des granulés, la granulation est achevée avec de l'eau purifiée.

Les granulés sont séchés dans un four à 40°C jusqu'à l'obtention d'une perte de 1-3 % et les granulés séchés sont calibrés sur un tamis de 0,8 mm.

Les ingrédients de la phase externe, à l'exception du stéarate de magnésium, c'est-à-dire le monohydrate de lactose de type Flowlac®, la cellulose microcristalline de type Avicel® PH 102 et le carmellose calcium, sont ajoutés aux granulés et mélangés avec un agitateur de type Turbula pendant 15 minutes. Le stéarate de magnésium, tamisé à 0,315 mm, est ajouté en dernier puis mélangé pendant 5 minutes.

Le mélange final est comprimé sur une machine à comprimer équipée de poinçons 12R12.

Enfin, un enrobage est préparé avec le mélange Opadry® White constitué de polyvinylalcool, dioxyde de titane, macrogol 3350 et de talc, afin de supprimer l'odeur et le goût du racécadotril.

### Exemple 3

Biodisponiblité comparée *in vivo* chez des volontaires humains sains

L'étude croisée à double aveugle a été conduite chez huit volontaires sains afin de comparer la biodisponibilité relative de la nouvelle formulation sous forme de comprimés de 175 mg de racécadotril à la formulation de référence (gélules de 100 mg de racécadotril).

Les comprimés ont été administrés deux fois par jour, le matin et le soir, et les gélules trois fois, le matin, à midi et le soir, selon les recommandations actuelles. Par conséquent, les patients ont reçu les comprimés et capsules à une dose totale de 350 mg par 24 heures et 300 mg par 24 heures respectivement. La biodisponibilité a été évaluée par mesure des taux de racécadotrilate, le métabolite actif du racécadotril dans le sérum sanguin, à différents moments sur une période de 24 heures. Pour cela, un test validé hautement spécifique et hautement sensible basé sur l'évaluation HPLC/MS de racécadotrilate prédérivatisé a été utilisé. Plusieurs observations ont indiqué que, de façon inattendue, la formulation sous forme de comprimés démontre un profil de biodisponibilité supérieur, à savoir :
- les valeurs Tmax moyennes, c'est-à-dire les périodes nécessaires pour observer le pic des taux sanguins après injection étaient de 1,15 heures et 1,66 heure pour les comprimés et les gélules, respectivement. Ceci est inattendu dans la mesure où les gélules permettent en général de rendre l'ingrédient actif biodisponible plus rapidement.
- les valeurs AUC (Aire sous la courbe) totales sur 24 heures, exprimées en nM.h, étaient de 4669 et 2552 pour les comprimés et gélules, respectivement, correspondant à des aires sous la courbe par mg de 13,34 et 8,50 respectivement. Cela indique que le racécadotril était plus disponible de 56 % lorsqu'il était formulé sous forme de comprimés que dans la gélule standard habituellement utilisée.
- la meilleure biodisponibilité des comprimés a permis ainsi une administration deux fois par jour en comparaison avec l'administration actuelle de trois fois par jour. En conséquence, les concentrations du sérum de racécadotrilate 12 heures après l'administration des comprimés (et immédiatement avant l'administration suivante) étaient encore suffisamment élevées (environ 4 nM) pour inhiber l'enzyme enképhalinase cible, qui présente une valeur Ki de 2 nM pour le métabolite actif.

### Exemple 4

### Effet clinique comparé dans la diarrhée sévère

Cette comparaison a été conduite dans le cadre d'une étude multicentrique, à double aveugle et double placebo sur 221 sujets adultes ambulatoires souffrant de diarrhée aiguë. Les patients ont reçu soit un comprimé de 175 mg deux fois par jour (110 patients) soit la gélule traditionnelle de 100 mg trois fois par jour (111 patients). Les critères d'inclusion correspondaient à l'apparition soudaine de diarrhée aiguë, définie par la présence d'au moins trois selles liquides ou semi-solides durant les dernières 24 heures et pendant une durée n'excédant pas 72 heures. Les critères de non inclusion comprenaient la présence de sang ou pus dans les selles ainsi qu'une diarrhée chronique.

Le critère d'évaluation principal correspondait au nombre de selles diarrhéiques ayant eu lieu entre le début du traitement et la guérison ou le jour 7 si les patients n'étaient pas guéris. Les critères additionnels incluaient le pourcentage de guérison lors de la visite finale, la durée de la diarrhée, l'évolution des symptômes associés, le besoin d'un traitement additionnel, le pourcentage de disparition de la diarrhée.

Les traitements ont débuté lors de la première visite ; les patients ont également été observés au jour 3 et au jour 7 et ont dû remplir un agenda décrivant leurs symptômes. Le nombre moyen de selles diarrhéiques avant guérison, c'est-à-dire le critère d'efficacité principal, était de 4 ± 3,8 pour les comprimés versus 6.2 ± 11.2 pour les gélules. La comparaison de ces valeurs au moyen d'une transformation racine carrée et ajustement de la sévérité de la diarrhée à la ligne de base a démontré que le traitement deux fois par jour avec les comprimés était, de façon inattendue, significativement supérieur à l'administration traditionnelle trois fois par jour avec la gélule (P ≤ 0,0001). Les critères secondaires ont montré les mêmes tendances et les seuls événements défavorables étaient mineurs, généralement liés à la pathologie plutôt qu'au traitement.

Plus particulièrement, l'analyse de la durée de la diarrhée a révélé que l'administration des comprimés deux fois par jour permettait de réduire significativement la durée de la diarrhée par rapport à l'administration de gélules trois fois par jour (13,73 h vs 17,48 h ; p= 0,0238).

Il peut donc être conclu que le nouveau traitement est plus efficace que le traitement traditionnel et qu'il démontre les avantages d'une administration plus aisée et d'une amélioration de l'observance du traitement, en particulier chez les patients qui ne veulent pas interrompre leur rythme journalier.

## Revendications

1. Comprimé de racécadotril constitué d'un noyau enrobé, ledit noyau comprenant du racécadotril, tel que le noyau comprend, en poids :
- 20 à 50 % de racécadotril ;
- 20 à 40 % de monohydrate de lactose ;
- 7 à 15 % de carmellose calcium ;
- 2 à 10 % d'hydroxypropylcellulose ;
- 5 à 10 % de cellulose microcristalline ;
- 2 à 10 % d'amidon prégélatinisé ;
- 0,5 à 5 % de stéarate de magnésium.

2. Comprimé de racécadotril selon la revendication 1 tel que ledit comprimé comprend entre 170 et 180 mg de racécadotril par comprimé.

3. Comprimé selon la revendication 1 ou 2 comprenant 175 mg de racécadotril par comprimé.

4. Comprimé selon l'une quelconque des revendications précédentes tel que le noyau comprend :
- 175 mg de racécadotril ;
- 144,1 mg de monohydrate de lactose ;
- 41 mg de carmellose calcium ;
- 18 mg d'hydroxypropylcellulose ;
- 32,5 mg de cellulose microcristalline;
- 25 mg d'amidon prégélatinisé ;
- 4,4 mg de stéarate de magnésium.

5. Procédé de préparation d'un comprimé de racécadotril selon l'une quelconque des revendications précédentes comprenant les étapes de :
1) la préparation du noyau comprenant le racécadotril, puis
2) l'enrobage dudit noyau.

6. Procédé selon la revendication 5 tel que l'étape 1) comprend :
(i) la granulation ;
(ii) le séchage des granules obtenus ;
(iii) l'ajout et le mélange de la phase externe ; et
(iv) la compression du mélange final.

7. Comprimé de racécadotril constitué d'un noyau enrobé, ledit noyau comprenant du racécadotril, pour son utilisation pour le traitement de la diarrhée, par administration dudit comprimé deux fois par jour.

8. Comprimé de racécadotril pour son utilisation selon la revendication 7 tel que ledit comprimé comprend entre 170 et 180 mg de racécadotril par comprimé.

9. Comprimé pour son utilisation selon la revendication 7 ou 8 comprenant 175 mg de racécadotril par comprimé.

10. Comprimé pour son utilisation selon l'une quelconque des revendications 7 à 9 comprenant de 20 % à 50 % en poids de racécadotril.

11. Comprimé pour son utilisation selon l'une quelconque des revendications 7 à 10 tel que ledit noyau comprend, en plus du racécadotril, un ou plusieurs excipients choisis parmi les charge(s), liant(s), désintégrant(s), lubrifiant(s).

12. Comprimé pour son utilisation selon la revendication 11 tel que la ou les charges sont choisies parmi le monohydrate de lactose, la cellulose microcristalline, le mannitol, le sorbitol.

13. Comprimé pour son utilisation selon la revendication 11 tel que le ou les liants sont choisis parmi l'hydroxypropylcellulose, la polyvidone.

14. Comprimé pour son utilisation selon l'une quelconque des revendications 7 à 13 tel que l'enrobage est constitué d'un ou plusieurs excipients choisis parmi les agents augmentant la viscosité, les opacifiants, les agents plastifiants hydrophiles et les agents opacifiants colorants.

15. Comprimé pour son utilisation selon l'une quelconque des revendications 7 à 14 tel que le noyau comprend, en poids :
- 20 à 50 % de racécadotril ;
- 25 à 50 % de charge(s) ;
- 9 à 25 % de désintégrant(s) ;
- 2 à 10 % de liant(s) ;
- 0,5 à 5 % de lubrifiant(s).

16. Comprimé pour son utilisation selon l'une quelconque des revendications 7 à 15 tel que le noyau comprend, en poids :
- 20 à 50 % de racécadotril ;
- 20 à 40 % de monohydrate de lactose ;
- 7 à 15 % de carmellose calcium ;
- 2 à 10 % d'hydroxypropylcellulose ;
- 5 à 10 % de cellulose microcristalline ;
- 2 à 10 % d'amidon prégélatinisé ;
- 0,5 à 5 % de stéarate de magnésium.

17. Comprimé pour son utilisation selon l'une quelconque des revendications 7 à 16 tel que le noyau comprend:
- 175 mg de racécadotril ;
- 144,1 mg de monohydrate de lactose ;
- 41 mg de carmellose calcium ;
- 18 mg d'hydroxypropylcellulose ;
- 32,5 mg de cellulose microcristalline;
- 25 mg d'amidon prégélatinisé ;
- 4,4 mg de stéarate de magnésium.

## Claims

1. Racecadotril tablet comprising a coated core, said core containing the racecadotril, wherein the core comprises, by weight:
- 20 to 50 % racecadotril;
- 20 to 40 % lactose monohydrate;
- 7 to 15 % calcium carmellose;
- 2 to 10 % hydroxypropyl cellulose;
- 5 to 10 % microcrystalline cellulose;
- 2 to 10 % pre-gelatinised starch;
- 0.5 to 5 % magnesium stearate.

2. Racecadotril tablet according to claim 1, wherein said tablet contains between 170 and 180 mg of racecadotril per tablet.

3. Tablet according to claim 1 or 2, containing 175 mg of racecadotril per tablet.

4. Tablet according to any one of the preceding claims, wherein the core contains:
- 175 mg of racecadotril;
- 144.1 mg of lactose monohydrate;
- 41 mg of calcium carmellose;
- 18 mg of hydroxypropyl cellulose;
- 32.5 mg of microcrystalline cellulose;
- 25 mg of pre-gelatinised starch;
- 4.4 mg of magnesium stearate.

5. Method of preparing a racecadotril tablet according to any one of the preceding claims, comprising the steps of:
1) preparing the core containing the racecadotril, then
2) coating said core.

6. Method according to claim 5, wherein step 1) involves:
(i) granulation
(ii) drying the granules obtained;
(iii) adding and mixing the external phase; and
(iv) compressing the final mixture.

7. Racecadotril tablet comprising a coated core, said core containing the racecadotril, for use thereof in the treatment of diarrhoea by administration of said tablet twice a day.

8. Racecadotril tablet for use thereof according to claim 7, wherein the tablet contains between 170 and 180 mg of racecadotril per tablet.

9. Tablet for use thereof according to claim 7 or 8, containing 175 mg of racecadotril per tablet.

10. Tablet for use thereof according to any one of claims 7 to 9 containing 20 % to 50 % by weight of racecadotril.

11. Tablet for use thereof according to any one of claims 7 to 10, wherein said core contains, in addition to racecadotril, one or more excipients selected from filler(s), binder(s), disintegrant(s), lubricant(s).

12. Tablet for use thereof according to claim 11, wherein the filler or fillers are selected from lactose monohydrate, microcrystalline cellulose, mannitol, sorbitol.

13. Tablet for use thereof according to claim 11, wherein the binders are selected from hydroxypropyl cellulose, polyvidone.

14. Tablet for use thereof according to any one of claims 7 to 13, wherein the coating is made from one or more excipients selected from agents for increasing viscosity, opacifiers, hydrophilic plasticisers and colouring opacifiers.

15. Tablet for use thereof according to any one of claims 7 to 14, wherein the core contains, by weight:
- 20 to 50 % racecadotril;
- 25 to 50 % filler(s);
- 9 to 25 % disintegrant(s);
- 2 to 10 % binder(s);
- 0.5 to 5 % lubricant(s).

16. Tablet for use thereof according to any one of claims 7 to 15, wherein the core contains, by weight:
- 20 to 50 % racecadotril;
- 20 to 40 % lactose monohydrate;
- 7 to 15 % calcium carmellose;
- 2 to 10 % hydroxypropyl cellulose;
- 5 to 10 % microcrystalline cellulose;
- 2 to 10 % pre-gelatinised starch;
- 0.5 to 5 % magnesium stearate.

17. Tablet for use thereof according to any one of claims 7 to 16, wherein the core contains:
- 175 mg of racecadotril;
- 144.1 mg of lactose monohydrate;
- 41 mg of calcium carmellose;
- 18 mg of hydroxypropyl cellulose;
- 32.5 mg of microcrystalline cellulose;
- 25 mg of pre-gelatinised starch;
- 4.4 mg of magnesium stearate.

## Patentansprüche

1. Racecadotriltablette, die aus einem umhüllten Kern besteht, wobei der Kern Racecadotril umfasst, wobei der Kern in Gewichtsprozent:
- 20 Gew.-% bis 50 Gew.-% Racecadotril,
- 20 Gew.-% bis 40 Gew.-% Laktosemonohydrat,
- 7 Gew.-% bis 15 Gew.-% Calciumcarmellose,
- 2 Gew.-% bis 10 Gew.-% Hydroxypropylcellulose,
- 5 Gew.-% bis 10 Gew.-% mikrokristalline Cellulose,
- 2 Gew.-% bis 10 Gew.-% vorgelatinierte Stärke,
- 0,5 Gew.-% bis 5 Gew.-% Magnesiumstereat umfasst.

2. Racecadotriltablette gemäß Anspruch 1, wobei die Tablette zwischen 170 mg und 180 mg Racecadotril pro Tablette umfasst.

3. Tablette gemäß Anspruch 1 oder 2, die 175 mg Racecadotril pro Tablette umfasst.

4. Tablette gemäß irgendeinem der vorhergehenden Ansprüche, wobei der Kern umfasst:
- 175 mg Racecadotril,
- 144,1 mg Laktosemonohydrat,
- 41 mg Calciumcarmellose,
- 18 mg Hydroxypropylcellulose,
- 32,5 mg mikrokristalline Cellulose,
- 25 mg vorgelatinierte Stärke,
- 4,4 mg Magnesiumstereat.

5. Verfahren zum Herstellen einer Racecadotriltablette gemäß irgendeinem der vorhergehenden Ansprüche, das die folgenden Schritte umfasst:
1) Herstellen des Racecadotril umfassenden Kerns, und anschließend
2) Umhüllen des Kerns.

6. Verfahren gemäß Anspruch 5, wobei Schritt 1) umfasst:
(i) die Granulation,
(ii) das Trocknen der erhaltenen Körnchen,
(iii) das Hinzufügen und Mischen der externen Phase,
und
(iv) das Komprimieren der Endmischung.

7. Racecadotriltablette, die aus einem umhüllten Kern besteht, wobei der Kern Racecadotril umfasst, zur Verwendung für das Behandeln von Durchfall durch Verabreichen der Tablette zweimal täglich.

8. Racecadotriltablette zur Verwendung gemäß Anspruch 7, wobei die Tablette zwischen 170 mg und 180 mg Racecadotril pro Tablette umfasst.

9. Tablette zur Verwendung gemäß Anspruch 7 oder 8, die 175 mg Racecadotril pro Tablette umfasst.

10. Tablette zur Verwendung gemäß irgendeinem der Ansprüche 7 bis 9, die 20 Gew.-% bis 50 Gew.-% Racecadotril umfasst.

11. Tablette zur Verwendung gemäß irgendeinem der Ansprüche 7 bis 10, wobei der Kern zusätzlich zum Racecadotril einen oder mehr Exzipienten umfasst, die aus Füllstoff(en), Bindemittel(n), Zerfallhilfsmittel(n) und Schmiermittel(n) ausgewählt werden.

12. Tablette zur Verwendung gemäß Anspruch 11, wobei der oder die Füllstoffe aus Laktosemonohydrat, mikrokristalliner Cellulose, Mannitol und Sorbit ausgewählt wird /werden.

13. Tablette zur Verwendung gemäß Anspruch 11, wobei das oder die Bindemittel aus Hydroxypropylcellulose und Polyvidon ausgewählt wird /werden.

14. Tablette zur Verwendung gemäß irgendeinem der Ansprüche 7 bis 13, wobei die Umhüllung aus einem oder mehr Exzipienten besteht, die aus Mitteln, die die Viskosität erhöhen, Trübungsmitteln, hydrophilen Weichmachern und färbenden Trübungsmitteln ausgewählt werden.

15. Tablette zur Verwendung gemäß irgendeinem der Ansprüche 7 bis 14, wobei der Kern in Gewichtsprozent:
- 20 Gew.-% bis 50 Gew.-% Racecadotril,
- 25 Gew.-% bis 50 Gew.-% Füllstoff(e),
- 9 Gew.-% bis 25 Gew.-% Zerfallhilfsmittel
- 2 Gew.-% bis 10 Gew.-% Bindemittel,
- 0,5 Gew.-% bis 25 Gew.-% Schmiermittel umfasst.

16. Tablette zur Verwendung gemäß irgendeinem der Ansprüche 7 bis 15, wobei der Kern in Gewichtsprozent:
- 20 Gew.-% bis 50 Gew.-% Racecadotril,
- 20 Gew.-% bis 40 Gew.-% Laktosemonohydrat,
- 7 Gew.-% bis 15 Gew.-% Calciumcarmellose,
- 2 Gew.-% bis 10 Gew.-% Hydroxypropylcellulose,
- 5 Gew.-% bis 10 Gew.-% mikrokristalline Cellulose,
- 2 Gew.-% bis 10 Gew.-% vorgelatinierte Stärke,
- 0,5 Gew.-% bis 5 Gew.-% Magnesiumstereat umfasst.

17. Tablette zur Verwendung gemäß irgendeinem der Ansprüche 7 bis 16, wobei der Kern
- 175 mg Racecadotril,
- 144,1 mg Laktosemonohydrat,
- 41 mg Calciumcarmellose,
- 18 mg Hydroxypropylcellulose,
- 32,5 mg mikrokristalline Cellulose,
- 25 mg vorgelatinierte Stärke,
- 4,4 mg Magnesiumstereat
umfasst.
